# EUROPEAN PATENT SPECIFICATION

(11) **EP 2 153 859 B1**
(45) Date of publication and mention of the grant of the patent: **23.12.2015**
(21) Application number: 08162308.4
(22) Date of filing: 13.08.2008
(51) Int. Cl.: A61M 16/20, A61M 16/08

(54) **Medical ventilation system**
Medizinisches Beatmungssystem
Système de ventilation médicale

(43) Date of publication of application: 17.02.2010
(73) Proprietor: Drägerwerk AG & Co. KGaA, 23558 Lübeck (DE)
(72) Inventor: van Weert, Marinus, 5626 HK, Vught (NL); van Dijk, Geert, 5325 XH, Well (NL)
(74) Representative: Mildner, Volker

(56) References cited:
- EP-A- 0 990 448
- WO-A-82/00766
- FR-A- 2 573 658
- US-A- 4 462 397
- US-A- 6 102 038
- US-A1- 2002 020 414

## Description

### FIELD OF THE INVENTION

The invention relates to a ventilation system, in particular a medical ventilation system.

### BACKGROUND OF THE INVENTION

In medical applications, mechanical ventilators are used to ventilate patients, for example in transfer situations, to control the patient's breathing. By monitoring the patient's spontaneous breathing activity, the ventilators may prevent that patients experience unpleasant controlled ventilation by actively supporting the patient's breathing. Also, it may be necessary to monitor the breathing of the patient to prevent apnea, for example.

Consequently, next to an inhalation gas supply which provides inhalation gas to a ventilation hose, the ventilator has connections for a flow sensor. These connections usually have the form of two connectors for connecting flow measurement hoses; pressures at different positions in the hoses are compared to obtain an estimate of the flow. Also, a means is provided in the ventilator for controlling the positive end expiration pressure (PEEP). Such a means may comprise an expiration valve and a connector for connecting a pressure control hose to control the expiration valve. The ventilator applies a pressure to the pressure control hose corresponding to a desired PEEP value. Alternatively, the ventilator does not have a separate connection for the control of the PEEP. Instead, the inhalation gas supply is used to control the PEEP by means of an expiration valve of which the pressure is controlled via the ventilation hose.

The inhalation gas is delivered to the patient via a ventilation hose. The mechanical parts used to measure the expiration flow and to impose the positive end expiration pressure are located at the patient side of the ventilation hose. This is for example the case in US 6,102,038, which discloses an adjustable exhalation valve assembly in which the closing function and the exhalation pressure control are combined in a single mechanism. Because the exhalation valve assembly is attached to a mask or to an endotracheal tube, which is in turn affixed to the patient's trachea, the weight and bulk of the exhalation valve assembly can at the least create patient discomfort, and at the worst can lead to extubation or tissue damage.

US 4,462,397 discloses a breathing circuit including a double-wall coaxial main tube and a manifold including two sets of coaxially disposed inner and outer manifold tubes positioned along the length of the coaxial main tube. The manifold is provided with a nebulizer, a bypass interconnecting the exhalation path of two outer tube sections which are closed and separated by the nebulizer, and an exhalation valve provided in the bypass path and including a valve body capable of closing the bypass during inhalation.

US 2002/0020414 A1 discloses a multilumen valve assembly for use in providing resuscitation and assisted ventilation. The assembly has a first and a second source conduit, an exhaust conduit, a one-way valve, and an exhaust valve. In one embodiment, the exhaust valve is closed when the one-way valve is open. In an alternative co-axial valve embodiment, a shunt extends between the first inspiratory conduit and a valve in the exhaust conduit. A corresponding coaxial conduit may be connected to the distal end of the second inspiratory conduit and distal end of the exhaust conduit.

WO 82/00766 discloses an anesthesia-breathing apparatus by means of which gas can be supplied to a patient for artificial respiration. The apparatus comprises a gas chamber connected to a patient connecting piece and, on the other hand, a respiratory gas receptacle in the form of a respiration bag, a check valve adapted to permit discharge of excess gas from the breathing apparatus to the atmosphere.

EP 0 990 448 A2 discloses a valve connectable to a common flow path for inspiration and expiration gas in a patient ventilation system to separate the flow paths of the inspiration and expiration gases. EP 0 990 448 A2 further discloses a differential pressure flow meter.

### SUMMARY OF THE INVENTION

It would be advantageous to have an improved ventilation system. To better address this concern, an adapter is presented for connecting a ventilation hose to a ventilator, the ventilation hose comprising at least a hose inhalation conduit, the adapter comprising
an adapter inhalation conduit having a first end arranged for connection to the hose inhalation conduit and a second end configured for connection to an inhalation gas supply of the ventilator;
an adapter exhalation conduit having a first end arranged for connection to a hose exhalation conduit and a second end openable to atmosphere; and
means for closing the second end for providing a positive end expiration pressure; and
wherein the adapter exhalation conduit comprises a flow sensor for providing a signal representing a flow of fluid arriving via the first end of the adapter exhalation conduit.

Using this adapter, the weight and bulk of the exhalation valve assembly is taken away from the mouth piece. Consequently, the weight at the patient is reduced, which has a positive effect on patient comfort and safety. The adapter allows to use an exhalation conduit to guide the exhalation gases away from the patient instead of providing means for providing a positive end expiration pressure near the mouth of the patient. The adapter allows to use such an exhalation conduit when a ventilator is used which does not support the use of an exhalation conduit.

The adapter may further provide a fluid communication between the adapter inhalation conduit and the second end of the adapter exhalation conduit for providing a control pressure for controlling the positive end expiration pressure. This allows to use the inhalation gas supply for controlling the positive end expiration pressure within the adapter.

The adapter comprises a flow sensor for measuring a flow of exhalation gases. Providing a flow sensor in the adapter removes the need to provide a flow sensor near the mouth piece. This further reduces the weight imposed on the mouth piece.

The adapter may provide a first connector for connecting the adapter inhalation conduit to the inhalation gas supply of the ventilator, and a second connector for connecting a channel arranged for carrying a signal produced by the flow sensor to a flow sensor connector of the ventilator. Preferably the first connector and the second connector have fixed relative positions. These fixed relative positions may coincide with relative positions of corresponding connectors of the ventilator. This allows the adapter to be connected to or disconnected from the ventilator in an efficient way.

The first end of the adapter exhalation conduit may have a fixed relative position with respect to the first end of the adapter inhalation conduit. These first ends may be arranged complementary to corresponding ends of the hose inhalation conduit and the hose exhalation conduit. This is particularly useful when using a single ventilation hose comprising both hose inhalation and exhalation conduits. It allows to easily and safely exchanging the hose and/or the adapter.

An embodiment comprises a valve for providing an openable closure of the second end of the adapter exhalation conduit, wherein the second end of a wall of the adapter exhalation conduit is chamfered where it reaches the valve. A reduced effective wall thickness leads to a larger contact pressure between the walls of the conduit and the valve, which results in an improved control of the positive end expiration pressure.

The present invention is defined by the independent claim 1. The dependent claims define advantageous embodiments.

### BRIEF DESCRIPTION OF THE DRAWINGS

These and other aspects of the invention will be further elucidated and described with reference to the drawing, in which
Fig. 1 is a diagram illustrating an exterior of a ventilator and several interfaces;
Fig. 2 is a diagram illustrating an interface connected to a ventilator;
Fig. 3 is a diagram illustrating an arrangement of a ventilator, an adapter, and a ventilation hose;
Fig. 4 is a diagram illustrating functional aspects of an adapter;
Fig. 5 shows an exterior of an adapter and a ventilation hose;
Fig. 6 shows a cross sectional view of an adapter and a ventilation hose;
Fig. 7 is a diagram illustrating an expiration/PEEP valve.

### DETAILED DESCRIPTION OF EMBODIMENTS

In this description, several embodiments of ventilator accessories and methods of operating ventilators and their accessories are described. These embodiments may be advantageously used in combination with a ventilator and one or more hoses providing at least an inhalation conduit (which provides gases for inhalation to the patient) and an exhalation conduit (which guides exhalation gases away from the patient). A two hose system may be used, which is characterized by the use of an inhalation hose and an exhalation hose. Although a two hose system has the advantage that there is no heavy valve and/or flow sensor close to the patient, the presence of two hoses is not very convenient for the patient. A coaxial hose has been developed, which reduces the number of hoses to one, while maintaining the advantages of a two hose system, because of the two conduits comprised in the coaxial hose. A coaxial hose is known from, for example, US4265235. Coaxial hoses are known to have a construction where the coaxial hose is split at the ventilator side, so that the conduits end in a non-coaxial way. The end of the outer lumen of the coaxial hose may be connected to a flexible, non-coaxial side tube diverging from the coaxial hose. Such an arrangement is shown in US2006/0162726.

For example, the inner conduit is used for the fresh gas going towards the patient side, and the outer conduit is for the expiration gases. The other end of these conduits is connected somehow to the ventilator inspiration connector and the ventilator expiration connector.

It is possible to connect a coaxial hose to a ventilator with only a connection for an inhalation hose and two separate flexible hoses for the flow sensor. In that case the inner conduit may be connected to the ventilator. The outer hose may be connected to an expiration valve and a flow sensor. As the expiration hose ends into a flexible diverging part, the valves and flow sensor can be connected to this part. A disadvantage of this construction is that, since the rather heavy parts are connected to a flexible hose, this can easily lead to disruption and/or disconnection of the hose.

US 6102038 describes a way of combining a PEEP valve with an exhalation valve. To be able to control the PEEP accurately, an additional hose from the ventilator to the PEEP valve may be provided. Such a construction reduces the weight at the patient side compared to a system with separate PEEP and exhalation valves. However using a coaxial hose, the weight at the patient side may be reduced further.

Using the techniques described herein, it is possible to use a dual conduit system such as a coaxial hose in combination with a standard ventilator being configured for use with a single ventilation conduit. An example of such a ventilator is the Oxylog 3000 manufactured by Dräger Medical BV, Best, The Netherlands. An adaptor piece may be provided between a coaxial hose and an inspiration connector of the ventilator. This may overcome some of the disadvantages of the prior art. The adapter, which may be a rigid object, may comprise the connection for the inspiration hose, a check valve to control the inspiration, a flow sensor, and a combined expiration/PEEP valve. The inspiration hose may be connected to the existing outlet on the ventilator via the adapter. Two flow measurement conduits may be used to connect the flow sensor with flow sensor connections on the ventilator. These two flow measurement conduits may be formed by two small hoses or by two rigid tubes, for example. If the adapter has a rigid construction holding the connectors which connect to the ventilator (inhalation conduit, flow measurement conduits), where the relative positions of the connectors of the adapter correspond to the relative positions of the corresponding outlets on the ventilator, it is easy to push the parts on the ventilator without the danger of damages. Also, one can make all connections in one movement. Instead of first making the connection with the inspiration hose and after that connect the two flow sensor hoses, the adapter piece enables to make the three connections with one push. This is very convenient for the medical personnel, especially when a change of hoses or a change of ventilator is carried out during an emergency situation.

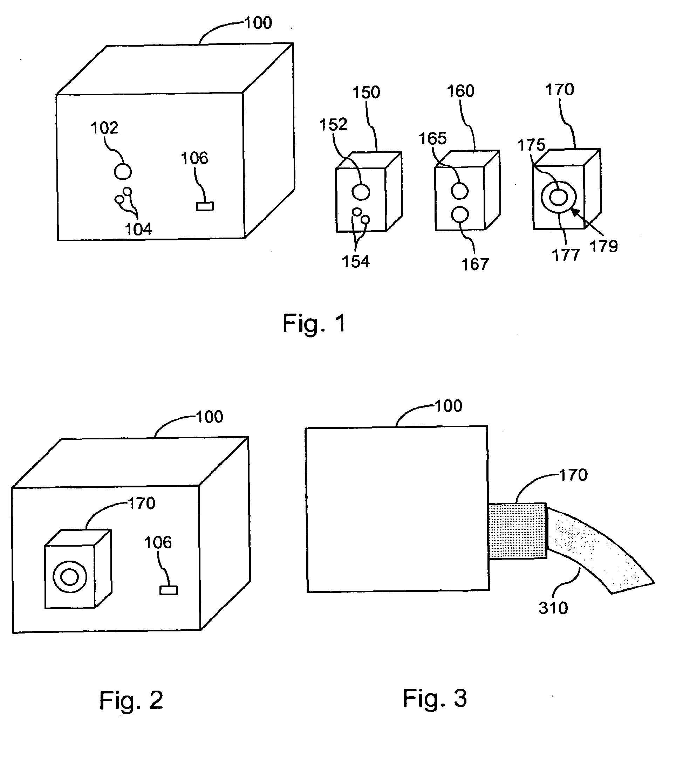

Fig. 1 illustrates schematically an exterior of a ventilator 100 which may be used for controlling the respiration of a patient. Only the aspects which are needed for explanation of the embodiments described herein are shown. The ventilator 100 comprises an inhalation gas supply connector 102. This gas supply is used, among others, for delivering the gas mixture which is to be supplied to the patient via a hose. The ventilator 100 further comprises two flow measurement connectors 104 for connection with two respective flow measurement hoses. Inside the ventilator, a means may be provided for calculating a flow of gas based on the pressure in each of the flow measurement hoses. The ventilator 100 may have other inlets, such as a power connector 106 for connection with a power supply. Other outlets, as well as control means and display means may be present in the ventilator, although these features are not drawn in the figure. Fig. 1 also illustrates a frontal view of an interface 150. The interface comprises an inhalation gas connector 152 which fits to the inhalation gas supply connector 102 of the ventilator 100. The interface further comprises two flow measurement connectors 154. The relative positions of the inhalation gas connector 152 and the flow measurement connectors 154 corresponds to the relative positions of the gas supply connector 102 and the flow measurement connectors 104. Also, the dimensions and shapes of the connectors are such that the connectors of the ventilator fit to the corresponding connectors of the interface. Consequently, the adapter 150 may be connected to the ventilator 100 in an efficient way, for example by properly positioning the adaptor next to the relevant connections of the ventilator and pushing the adapter such that the connectors 152, 154 of the adapter 100 are fixed to the corresponding connectors 102, 104 of the ventilator. Fig. 1 also shows a rear view 160 of the adapter 150. The rear side of the adapter comprises an inhalation hose connector 165 for connection of an inhalation hose which guides the gases provided by the inhalation gas supply connector 102 via connectors 152 and 165 to the patient. The adapter further comprises an exhalation hose connector 167 for connecting an exhalation hose to the adapter. The exhalation hose guides the exhalation gases from the patient to the exhalation hose connector 167. Inside the adapter, a means is provided for releasing the exhalation gases from the exhalation hose. Fig. 1 also shows a rear view of an alternative embodiment 170 of the adapter. The frontal side of the alternative embodiment of the adapter is identical to that of adapter 150. The rear side of the adapter 170 has a connector 179 for connecting a coaxial hose. An inner conduit 175 is used for transporting inhalation gases, while an outer conduit 177 is used for transporting exhalation gases. Alternatively, the inner conduit 175 is used for transporting exhalation gases and the outer conduit 177 is used for transporting inhalation gases.

Fig. 2 illustrates an arrangement in which the adapter 170, which has on its frontal side a plurality of connectors 152 and 154 as shown in Fig. 1, is connected to the ventilator. It is illustrated that the adapter 170 is fixed to the ventilator at the position of the connectors 102 and 104. Fig. 3 shows a side view of the ventilator 100, with an adapter 170 connected to it. A hose 310 is connected to the adapter 170.

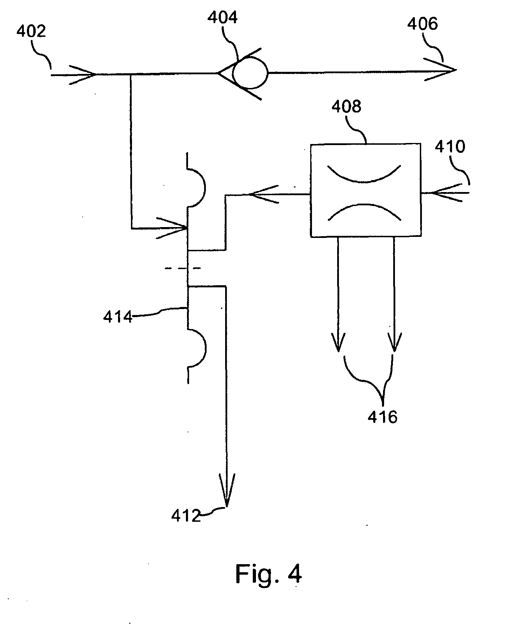

Fig. 4 is a functional diagram of an adapter 150. The adapter may have a connection 402 to an inhalation gases supply of a ventilator. If the pressure of the gases received from the ventilator opens the check valve 404, the received gases are forwarded via check valve 404 to a connection 406 of an inspiration conduit of a ventilation hose. The pressure of the received gases may also be used to control a membrane 414 which is used to realize a positive end expiration pressure in the patient exhalation gases circuit. However, other ways of providing a positive end expiration pressure may also be used. Expiration gases are received at 410 from an exhalation conduit of a ventilation hose. These exhalation gases may pass through an optional flow sensor 408. Such a flow sensor 408 may comprise an obstruction to obtain a pressure drop which may be measured, as known in the art. In such a case, conduits may be provided in fluid connection to respective locations proximal and distal of the obstruction, which lead to connections 416 of pressure measurement means. Typically such connections 416 are connected to a ventilator which comprises the pressure measurement means. Other kinds of flow sensors may also be used. For example, a flow sensor which generates an electrical signal may be used. In such a case, the electrical signal is provided to the pressure measurement means 416. After passing the flow sensor 408, and if the pressure of the exhalation gases is above a threshold which may be determined by the membrane 414 and by a control pressure from the inhalation gas supply 402, the exhalation gases are released to atmosphere at 412.

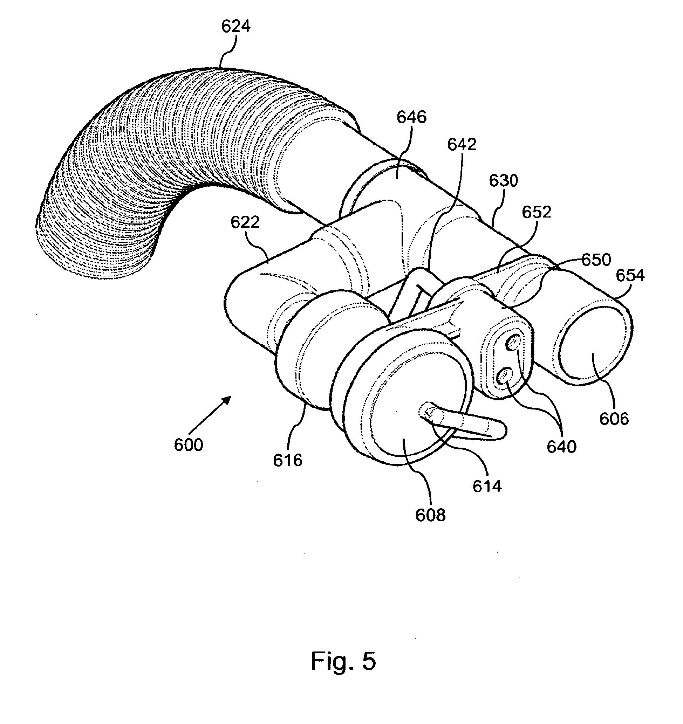

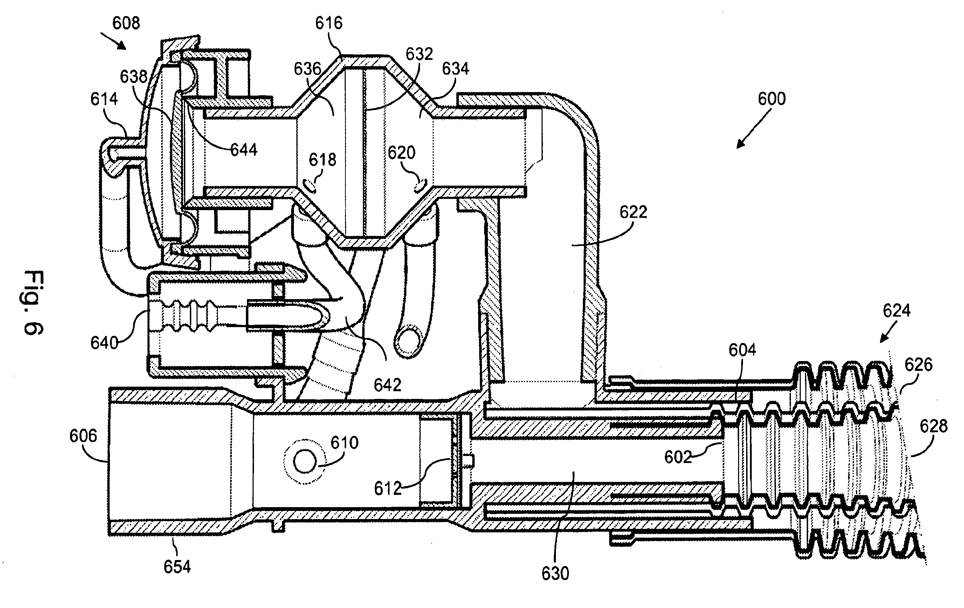

Fig. 5 shows an exterior view of an adapter 600 connected to a coaxial ventilation hose 624. Fig. 6 shows a cross sectional interior view of the adapter 600 and the coaxial ventilation hose 624. Although these figures and the description focus on adapters for a coaxial ventilation hose, it will be understood that it is also possible to use separate inhalation and exhalation hoses (as adapter 160 shown in figure 1). The corresponding connectors of the adapter could be implemented accordingly to fit to the hose used. Also, another way of combining an inhalation conduit and exhalation conduit in a single hose is possible, for example a hose comprising a longitudinal cross sectional wall could be used to create two conduits in a single hose.

Such an adapter 600 may be used for connecting a ventilation hose 624 to a ventilator, as the adapter 170 as shown in Fig. 3. The ventilation hose 624 comprises at least a hose inhalation conduit 628. The adapter comprises an adapter inhalation conduit 630. The adapter inhalation conduit 630 has a first end 602 arranged for connection to the hose inhalation conduit 628 of the ventilation hose 624, and a second end 606 arranged for connection to an inhalation gas supply 102 of the ventilator 100. The adapter 600 further comprises an adapter exhalation conduit 622. The adapter exhalation conduit 622 has a first end 604 arranged for connection to a hose exhalation conduit 626 and a second end 608 openable to atmosphere, for releasing exhalation gases. The adapter 600 further comprises means 638 for closing the second end during inspiration and for providing a positive end expiration pressure. Means 638 may comprise a valve, a membrane, or any other means for providing a positive end expiration pressure, as known in the art by itself.

The adapter exhalation conduit 622 comprises a flow sensor 616 for providing a signal representing a flow of fluid arriving via the first end 604 of the adapter exhalation conduit 622. This signal may have the form of gases having a particular pressure. The flow sensor 616 may comprise an obstruction 632 which causes different pressure in a space 634 proximal to the obstruction and a space 636 distal of the obstruction. A wall of the conduit may have an opening 620 in the space 634 proximal to the obstruction and an opening 618 in the space 636 distal to the obstruction. A conduit (a tube 642, for example) is in fluid communication with the space 636 distal to the obstruction via the opening 618. On the other side, the tube 642 ends in a connector 640 which allows the tube 642 to be connected to a flow sensor connector of a ventilator. A second connector 640 is provided and is connected via a conduit which ends at opening 620. Alternative embodiments of the flow sensor 616 may need only one connector 640, for example an electrical connector conveying an electrical signal.

The flow connectors 640 may be in fixed relative position to each other, by incorporating the connectors 640 in a piece of rigid material 650. This allows to quickly and efficiently connect both flow connectors to the ventilator in a single movement.

An inhalation gas supply connector 654 is provided for connecting the second end 606 of the adapter inhalation conduit 630 to the ventilator 100. This inhalation gas supply connector 654 may be made to stay in fixed relative position to the flow connectors 640, for example by providing a rigid object 652 on which these connectors 654, 640 are mounted. This may allow to connect the connectors 654, 640 in one movement to corresponding connectors on the ventilator.

In an embodiment, the walls of the adapter inhalation conduit 630, the walls of the adapter exhalation conduit 622, and a channel 642 providing the communication between the flow sensor and the flow connector, are substantially rigid. This makes the adapter more rigid and robust.

In an embodiment, an outside surface encapsulating at least the walls of the adapter inhalation conduit 630 and the flow sensor conduits 642 is provided. This makes the adapter more robust.

The adapter inhalation conduit 630 may comprise a check valve 612 configured for allowing a fluid to pass in a direction towards the first end 646 of the adapter inhalation conduit 630. The check valve 612 is arranged to substantially block the passage of gases in a direction towards the second end 606 of the adapter inhalation conduit 630.

The wall of the adapter inhalation conduit 630 may have an opening 610 between the check valve 612 and the second end 606 of the adapter inhalation conduit 630. This opening 610 provides a fluid communication with the means 638 for closing the second end 608 of the adapter exhalation conduit 622. This way, a control pressure can be provided via the second end 606 of the inhalation conduit 630 to the means 638, which allows to keep it closed during inspiration and to control the positive end expiration pressure.

In an embodiment, the first end 604 of the adapter exhalation conduit 622 has a fixed relative position with respect to the first end 602 of the adapter inhalation conduit 630. In this embodiment, the adapter 600 comprises means for being connected to a ventilation hose 624 comprising both the hose inhalation conduit 628 and the hose exhalation conduit 626. The fixed relative position allows to efficiently connect and disconnect the ventilation hose 624 to and from the adapter 600.

In an embodiment, the means 614, 638 for closing the second end 608 of the adapter exhalation conduit 622 comprises a valve 638 for providing an openable closure of the second end of the adapter exhalation conduit, wherein the wall of the adapter exhalation conduit 622 at the second end 608 of the adapter exhalation conduit 622 is chamfered 644 where it reaches the valve 638.

An embodiment comprises a ventilation hose 624 comprising at least a hose inhalation conduit 628 and a separate hose exhalation conduit 626, the ventilation hose 624 being integrated with an adapter 600 as set forth.

With reference to Fig. 3, an embodiment is described which comprises a ventilation arrangement comprising a ventilator 100, a ventilation hose 310 comprising at least a hose inhalation conduit and a separate hose exhalation conduit. In this arrangement, the ventilation hose 310 is connected to the ventilator 100 by means of an adapter 170, as set forth in relation of Fig. 5 and Fig. 6.

To use the adapter, the first end of the adapter inhalation conduit may be connected to the hose inhalation conduit. The second end of the adapter inhalation conduit may be connected to the inhalation gas supply of the ventilator. The first end of the adapter exhalation conduit may be connected to the hose exhalation conduit.

If the ventilation hose 310 or 624 is sufficiently long, for example at least one meter, the valve for positive end expiration pressure and/or the flow sensor can be placed relatively far away from the patient, which relieves the patient from the weight of these items near his or her mouth.

Furthermore disclosed is a method of operating a ventilator using a ventilation hose comprising at least a hose inhalation conduit and a separate hose exhalation conduit. The method comprises connecting a first end of an adapter inhalation conduit 630 to the hose inhalation conduit 628. The method further comprises connecting a second end 606 of the adapter inhalation conduit 630 to an inhalation gas supply 102 of the ventilator 100. The method further comprises connecting a first end 604 of an adapter exhalation conduit 622 to the hose exhalation conduit 626. The adapter exhalation conduit 622 may have a second end 608 openable to atmosphere and closable for providing a positive end expiration pressure.

In the following, a more specific embodiment will be described, with reference to Fig. 5 and Fig. 6 in particular. Many of the features of this embodiment are optional. Fig. 5 illustrates an adapter 600 connected to a coaxial hose 624. The coaxial hose in the figure may be of a known type. The basic principle of the adapter does not depend on the exact construction of the coaxial hose. Inside the inspiration channel 630 of the adapter a check valve 612 is placed. This check valve 612 is used to separate the pressure for the PEEP control (614, 638) from the patient pressure during expiration. At the same time it prevents that the expiration of the patient enters the ventilator. A standard check valve can be used for this function as known in the art. The end piece 606 of the inspiration channel may be made of plastic and may have a standardized conical shape. This is done to enable a good fit with the connector 102 on the ventilator 100. An alternative is to make this part of a flexible rubber or a soft plastic, although this may make it more difficult to push the adapter on the ventilator.

The expiration part 622 may be connected to the coaxial hose via a kind of T-piece, as shown in Figs. 5 and 6. The exact shape and construction of this T-piece can be adapted according to any desired resistance properties in the expiration channel. The flow sensor and the expiration/PEEP-valve may be placed parallel to the inspiration channel. This allows to keep the dimensions of the adapter as a whole relatively small. The working principle of the flow sensor and the expiration/PEEP-valve may be of a conventional type. The expiration/PEEP-valve gets its pressure via a pilot line connected to the inspiration channel. The pilot line may be a flexible hose connecting the expiration/PEEP-valve to the inspiration channel at a location between the ventilator end and the check valve. Instead of a flexible hose the connection between the expiration/PEEP-valve and the inspiration channel can be made with a rigid connection. This may result in a more robust construction. Also, the cost of manufacturing may be reduced if using a rigid connection. For example, the pilot tube and the inhalation conduit of the adapter may be molded as a single object. The dimensions and shapes of the connection between the expiration/PEEP-valve and the inspiration channel may be used to obtain a desired resistance towards the expiration/PEEP-valve.

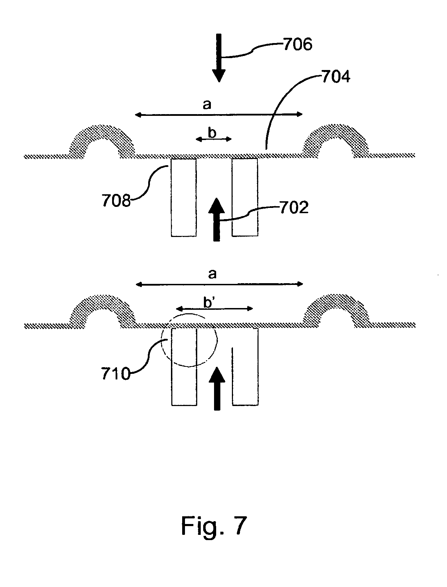

Fig. 7 illustrates two embodiments of the end of the exhalation conduit of the adapter and the expiration/PEEP-valve. The upper part of the figure illustrates a first embodiment of the expiration/PEEP-valve. The expiration pressure (in the direction indicated by arrow 702) pushes against the flexible membrane 704. The PEEP control-pressure controlled via the pilot line pushes against the expiration pressure valve 704 in the direction of arrow 706. The walls of the expiration conduit may be straight until end 708 of the conduit at a membrane forming the expiration valve. The lower part of Fig. 7 illustrates a second embodiment of the expiration/PEEP-valve. Most elements of this embodiment are similar to the first embodiment. However, the end 710 of the wall of the exhalation conduit is chamfered. The chamfer angle influences the working range of the PEEP-control. The small effective wall thickness obtained by virtue of the chamfered wall leads to a larger contact pressure, which allows to better control the expiration/PEEP-valve by means of the pilot line. In an embodiment, it is possible to exchange the expiration/PEEP-valve with another that has a differently chamfered end and has a corresponding different wall thickness and contact pressure. Such an embodiment allows to obtain a PEEP-range which is tailored to the PEEP control concept or the algorithm used to control the PEEP.

The two connectors for the flow sensor may be arranged in a block of material that is rigidly connected with the inspiration channel. This makes it possible to push all connectors on the ventilator at the same time. In the construction as it is shown in Fig. 6 there are two small hoses between the connector block and the flow sensor. This approach is chosen to support a flow sensor that senses the pressure before and after an obstruction to measure the flow. It is possible to integrate these small flow hoses as rigid tubes in the adapter. Such a construction would reduce the number of parts needed and make the adapter including the flow sensor conduits easier to manufacture.

Preferably, the distances between the flow sensor connectors and the inspiration channel connector of the adapter match with the distances between corresponding outlets of the ventilator. If these distances and the dimensions of the connectors are well designed, there is no disturbing leakage of gases at the connections. However, a slight deviation in these distances due to tolerance of the various parts might give rise to a leakage, either in the flow sensor lines or in the inspiration channel or both. To avoid any leakage, the connectors of the adapter are constructed as a substantially plastic part. Moreover, the connections to the flow sensor may each have a flexible hose inside. This flexible hose may be made of rubber or another flexible material. The interior surface of these flexible hoses may have small round ridges or fins to ensure a tight fit around the male connectors of the ventilator, thereby closing any gap in between the male connectors of the ventilator and the female connectors of the adapter. It will be understood that, if the ventilator has female connectors and the adapter has male connectors, these flexible tubes may be provided inside the ventilator connectors. Alternatively, similar flexible tubes having ridges on the outside surface may be provided around the male connectors. Such a construction is effective to take care of the tolerances and results in a connection without leakage. For an optimum connection of the flow sensor it is better to make the male connectors for the flow sensor (usually on the ventilator) a few millimeters longer than the male connector for the inspiration connector.

The expiration/PEEP-valve and the flow sensor are usually rather costly. In the solution presented in this description the rigid construction of the adapter makes it possible to reduce the number of parts considerably. That allows to reduce the material costs and/or the assembly costs. One can thus imagine that not only the hose is disposable, but also the adapter. This has the advantage that contamination between patients is less, since the contaminated expiration gases from the patient are going through a disposable part.

Another possibility is to make the adapter from a material that can withstand the conditions of sterilization. Since the valves and the flow sensor are not close to the patient, the weight of the material is less important. Consequently, other material choices can be made. By using materials that can withstand sterilization to construct the adapter, the adapter can be made reusable, possibly in combination with a disposable hose. Accordingly, the adapter may be sterilized; after sterilization, the adapter may be used with another patient.

Although in this description, the focus has been on a coaxial hose, it will be understood that any hose with two lumens can be used; one lumen to provide an inhalation hose conduit and another lumen to provide an exhalation hose conduit. For example, a hose can comprise two parallel lumens separated by an inner wall. Also, two separate hoses may be used. The adapter may form an integral part of the hose. Alternatively, the adapter and the hose may be constructed as separate parts which may be connected to and disconnected from each other. The patient and/or the medical personnel may experience the advantages of a two-way ventilation system although the ventilator does not need to have an exhalation gases outlet. This also may help preventing contamination of the ventilator with gases from the patient.

Ventilator systems can be based on a dual hose system or a single hose system. A ventilator with a dual hose system may be used in Critical Care departments of Hospitals, for example, since such a system gives more control over the ventilation performance than a single hose system. The single hose system is mainly used in non-invasive applications.

The dual hose system has two hoses: an inspiration hose and an expiration hose. The air, if necessary with an increased Oxygen percentage, is put under pressure via the inspiratory hose (first hose) into the patient. In the expiration phase the patient expires into the second hose into the ventilator. The expiration valve is inside the ventilator and is controlled by the ventilator. This expiration valve also controls the PEEP. The advantage of such a system is that the ventilation performance can be controlled very well. Furthermore there is little weight close to the patient, which is convenient. A disadvantage is more complexity and space necessary inside the ventilator. Furthermore there are two hoses going to the patient instead of one.

A single hose system has only an inspiration hose. This hose is used to bring the air into the patient. The connection at the patient contains also the expiration valve. The PEEP is therefore also controlled close to the patient. Furthermore the flow measurement has to be done close to the patient to cover the inspiratory flow as well as the expiratory flow. The advantage is that there is only one hose going to the patient. The disadvantage is that there is a rather bulky device close to the patient. Especially for invasive use this is not preferred.

The aim of the PEEP (Positive Expiratory End Pressure) control is to have a positive pressure during expiration. This usually improves the oxygenation of the patient. Basically the PEEP is controlled via a certain resistance in the expiration line. The most practical way to do it is to use the expiration valve. A certain pressure is put on this valve, such that it opens with a threshold pressure. This pressure can be realized via e.g. a spring. The advantage of a spring is that it does not need any additional control, but can be integrated into the expiration valve assembly. A disadvantage of a spring is that it can only be adjusted manually and furthermore the accuracy usually is poor.

A dual hose system has only two hoses between the patient and the ventilator. The PEEP adjustment is done inside the ventilator, as well as the flow measurement. At the patient there is only a Y-piece.

A single hose system has only an inspiration hose to the system. The flow measurement is done close to the patient and two additional small diameter hoses are needed for this. This placement of the flow meter gives the possibility to measure inspiratory and expiratory flow. If only the inspiratory flow needs to be measured, it can be done inside the ventilator and the two small diameter hoses are not necessary. The PEEP can be realized with a spring type approach, but this is not very reliable. Therefore an additional small hose is used between the patient and the ventilator for the PEEP control. It is possible to use the inspiration hose for this purpose and in that case the small hose is not necessary.

The ventilator may be either based on a dual hose system or on a single hose system. That means that either there are two respiration ports (inhalation and exhalation) or one respiration port (inhalation) with two small additional ports for the flow sensor on the ventilator. Considering the difference between the two systems the normal procedure is to make a choice for either a dual hose system or a single hose system. Ventilators which are used mainly as transport equipment or emergency equipment are usually based on a single hose system. The time that the patient is at this ventilator is usually short and a detailed control of the ventilation performance is therefore not so important. Furthermore such a ventilator is advantageously suitable for invasive and non-invasive ventilation.

Another application is a ventilator which is used during transport of patients. This can be transport from one hospital to another hospital, or inside the hospital from one department to another department. That means that the patient is disconnected from a fixed ventilator (which may be a dual hose system), and connected to a transport ventilator (which may be a single hose system). At the place of destination the transport ventilator is decoupled again and the patient is connected to a fixed ventilation system in that (hospital) room. However, it would be advantageous in such a situation to exchange only the ventilator and not the hoses. This would be especially advantageous if the hoses have identification tags which identify the patient with whom the hose is being used. If the patient can keep using the same hose, even when switching to a different kind of ventilator, this would be beneficial for the patient. In this case, the embodiments presented in this description can be used to make it possible to connect two hoses (or a dual-conduit hose) to a single-hose transport ventilator. Advantageously, the transport ventilator recognizes the patient based on the identification tag on the hose, and automatically can adjust itself according to settings specific to this patient, for example based on ventilation settings used for this patient by the fixed ventilator, for example.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. In the device claim enumerating several means, several of these means may be embodied by one and the same item of hardware. The mere fact that certain measures are recited in mutually different dependent claims does not indicate that a combination of these measures cannot be used to advantage.

## Claims

1. An adapter (600) for connecting a ventilation hose (624) to a ventilator (100), the ventilation hose (624) comprising at least a hose inhalation conduit (628), the adapter (600) comprising
an adapter inhalation conduit (630) having a first end (602) arranged for connection to the hose inhalation conduit (628) of the ventilation hose (624) and a second end (606) arranged for connection to an inhalation gas supply (102) of the ventilator (100); and
an adapter exhalation conduit (622) having a first end (604) arranged for connection to a hose exhalation conduit (626) and a second end (608) openable to atmosphere; and
means (638) for closing the second end (608) for providing a positive end expiration pressure;
**characterized by** the adapter exhalation conduit (622) comprising a flow sensor (616) for providing a signal representing a flow of fluid arriving via the first end (604) of the adapter exhalation conduit (622).

2. The adapter (600) according to claim 1, further comprising:
a flow connector (640) in communication with the flow sensor (616) and arranges for connection to the ventilator (100) and arranged for conveying the signal to the ventilator (100); and
an inhalation gas supply connector (654) in fixed relative position to the flow connector (640) and arranged for connecting the second end (606) of the adapter inhalation conduit (630) to the ventilator (100).

3. The adapter (600) according to claim 2, wherein the adapter inhalation conduit (630), the adapter exhalation conduit (622), and a channel (642) providing the communication between the flow sensor (616) and the flow connector (640) are substantially rigid.

4. The adapter (600) according to claim 3, comprising an outside surface encapsulating at least the adapter inhalation conduit (630) and the channel (642).

5. The adapter (600) according to claim 1, wherein the adapter inhalation conduit (630) comprises
a check valve (612) configured for allowing a fluid to pass in a direction towards the first end (602) of the adapter inhalation conduit (630); and
an opening (610) between the check valve (612) and the second end (606) of the adapter inhalation conduit (630) arranged for being in fluid communication with the means (638) for closing the second end (608) of the adapter exhalation conduit (622) for providing a control pressure for controlling the positive end expiration pressure.

6. The adapter (600) according to claim 1, wherein the first end (604) of the adapter exhalation conduit (622) has a fixed relative position with respect to the first end (602) of the adapter inhalation conduit (630), the adapter (600) comprising means for being connected to a ventilation hose comprising both the hose inhalation conduit (628) and the hose exhalation conduit (626).

7. The adapter (600) according to claim 6, wherein the means for being connected to a ventilation hose (624) is arranged for being connected to a coaxial hose.

8. The adapter (600) according to claim 1, wherein the means (614, 638) for closing the second end (608) of the adapter exhalation conduit (622) comprises a valve (638) for providing an openable closure of the second end (608) of the adapter exhalation conduit (622), wherein the second end (608) of the adapter exhalation conduit (622) is chamfered (644) where it reaches the valve (638).

9. A ventilation hose (624) comprising at least a hose inhalation conduit (628) and a separate hose exhalation conduit (626), the ventilation hose (624) being integrated with an adapter (600) according to any one of the previous claims.

10. A ventilation arrangement comprising a ventilator (100), a ventilation hose (624) comprising at least a hose inhalation conduit (628) and a separate hose exhalation conduit (626), the ventilation hose (624) being connected to the ventilator (100) by means of an adapter (600) according to any one of claims 1 to 8.

## Patentansprüche

1. Zwischenstück (600) zum Verbinden eines Gebläseschlauchs (624) mit einem Gebläse (100), wobei der Gebläseschlauch (624) zumindest eine Schlauchinhalationsleitung (628) umfasst, das Zwischenstück (600) umfassend:
eine Adapterinhalationsleitung (630) mit einem ersten Ende (602), das zur Verbindung mit der Schlauchinhalationsleitung (628) des Gebläseschlauchs (624) angeordnet ist, und einem zweiten Ende (606), das zur Verbindung mit einer Inhalationsgaszufuhr (102) des Gebläses (100) angeordnet ist; und
eine Adapterexhalationsleitung (622) mit einem ersten Ende (604), das zur Verbindung mit einer Schlauchexhalationsleitung (626) angeordnet ist, und einem zweiten Ende (608), das zu Atmosphäre geöffnet werden kann; und
Mittel (638) zum Schließen des zweiten Endes (608) zum Vorsehen eines positiven Endexpirationsdrucks;
**dadurch gekennzeichnet, dass** die Zwischenstückexhalationsleitung (622) einen Durchflusssensor (616) zum Vorsehen eines Signals umfasst, das einen Fluidfluss darstellt, der über das erste Ende (604) der Zwischenstückexhalationsleitung (622) ankommt.

2. Zwischenstück (600) nach Anspruch 1, ferner umfassend:
ein Flussverbindungsstück (640) das mit dem Durchflusssensor (616) in Verbindung steht und zur Verbindung mit dem Gebläse (100) angeordnet ist und zum Übermitteln des Signals zum Gebläse (100) angeordnet ist; und
einen Inhalationsgasverbinder (654) in starrer relativer Position zum Flussverbinder (640), der zum Verbinden des zweiten Endes (606) der Zwischenstückinhalationsleitung (630) mit dem Gebläse (100) angeordnet ist.

3. Zwischenstück (600) nach Anspruch 2, wobei die Zwischenstückinhalationsleitung (630), die Zwischenstückexhalationsleitung (622) und ein Kanal (642), der die Verbindung zwischen dem Durchflusssensor (616) und dem Flussverbinder (640) vorsieht, im Wesentlichen starr sind.

4. Zwischenstück (600) nach Anspruch 3, umfassend eine Außenfläche, die zumindest die Zwischenstückinhalationsleitung (630) und den Kanal (642) einkapselt.

5. Zwischenstück (600) nach Anspruch 1, wobei die Zwischenstückinhalationsleitung (630) Folgendes umfasst:
ein Rückschlagventil (612), das zum Ermöglichen konfiguriert ist, dass ein Fluid in einer Richtung zum ersten Ende (602) der Zwischenstückinhalationsleitung (630) durchläuft; und
eine Öffnung (610) zwischen dem Rückschlagventil (612) und dem zweiten Ende (606) der Zwischenstückinhalationsleitung (630), die zur Fluidverbindung mit dem Mittel (638) zum Schließen des zweiten Endes (608) der Zwischenstückexhalationsleitung (622) zum Vorsehen eines Regeldrucks zum Regeln des positiven Endexpirationsdrucks angeordnet ist.

6. Zwischenstück (600) nach Anspruch 1, wobei das erste Ende (604) der Zwischenstückexhalationsleitung (622) eine starre relative Position bezüglich des ersten Endes (602) der Zwischenstückinhalationsleitung (630) aufweist, wobei das Zwischenstück (600) Mittel zum Verbinden mit einem Gebläseschlauch umfasst, der sowohl die Schlauchinhalationsleiturig (628) als auch die Schlauchexhalationsleitung (626) umfasst.

7. Zwischenstück (600) nach Anspruch 6, wobei das Mittel zum Verbinden mit einem Gebläseschlauch (624) zum Verbinden mit einem koaxialen Schlauch angeordnet ist.

8. Zwischenstück (600) nach Anspruch 1, wobei das Mittel (614, 638) zum Schließen des zweiten Endes (608) der Zwischenstückexhalationsleitung (622) ein Ventil (638) zum Vorsehen eines zu öffnenden Verschlusses des zweiten Endes (608) der Zwischenstückexhalationsleitung (622) umfasst, wobei das zweite Ende (608) der Zwischenstückexhalationsleitung (622) dort abgefast (644) ist, wo es das Ventil (638) erreicht.

9. Gebläseschlauch (624), umfassend zumindest eine Schlauchinhalationsleitung (628) und eine separate Schlauchexhalationsleitung (626), wobei der Gebläseschlauch (624) mit einem Zwischenstück (600) nach einem der vorhergehenden Ansprüche integriert ist.

10. Gebläseanordnung, umfassend ein Gebläse (100), einen Gebläseschlauch (624), der zumindest eine Schlauchinhalationsleitung (628) und eine separate Schlauchexhalationsleitung (626) umfasst, wobei der Gebläseschlauch (624) mittels eines Zwischenstücks (600) nach einem der Ansprüche 1 bis 8 mit dem Gebläse (100) verbunden ist.

## Revendications

1. Adaptateur (600) destiné à connecter un tuyau de ventilation (624) à un respirateur (100), le tuyau de ventilation (624) comprenant au moins un conduit d'inhalation de tuyau (628), l'adaptateur (600) comprenant
un conduit d'inhalation d'adaptateur (630) ayant une première extrémité (602) prévue pour une connexion au conduit d'inhalation de tuyau (628) du tuyau de ventilation (624) et une deuxième extrémité (606) prévue pour une connexion à une alimentation de gaz d'inhalation (102) du respirateur (100), et
conduit d'exhalation d'adaptateur (622) ayant une première extrémité (604) prévue pour une connexion à un conduit d'exhalation de tuyau (626) et une deuxième extrémité (608) pouvant être ouverte vers l'atmosphère, et
un moyen (638) destiné à fermer la deuxième extrémité (608) pour fournir une pression expiratoire d'extrémité positive,
**caractérisé par le fait que** le conduit d'exhalation d'adaptateur (622) comprend un capteur d'écoulement (616) destiné à fournir un signal représentant un écoulement de fluide arrivant par le biais de la première extrémité (604) du conduit d'exhalation d'adaptateur (622).

2. Adaptateur (600) selon la revendication 1, comprenant en outre :
un connecteur d'écoulement (640) en communication avec le capteur d'écoulement (616) et prévu pour une connexion au respirateur (100) et prévu pour acheminer le signal vers le respirateur (100), et
un connecteur d'alimentation de gaz d'inhalation (654) dans une position relative fixe par rapport au connecteur d'écoulement (640) et prévu pour une connexion de la deuxième extrémité (606) du conduit d'inhalation d'adaptateur (630) au respirateur (100).

3. Adaptateur (600) selon la revendication 2, dans lequel le conduit d'inhalation d'adaptateur (630), le conduit d'exhalation d'adaptateur (622), et un canal (642) permettant la communication entre le capteur d'écoulement (616) et le connecteur d'écoulement (640) sont essentiellement rigides.

4. Adaptateur (600) selon la revendication 3, comprenant une surface extérieure encapsulant au moins le conduit d'inhalation d'adaptateur (630) et le canal (642).

5. Adaptateur (600) selon la revendication 1, dans lequel le conduit d'inhalation d'adaptateur (630) comprend
un clapet anti-retour (612) configuré pour permettre à un fluide de passer dans une direction orientée vers la première extrémité (602) du conduit d'inhalation d'adaptateur (630), et
une ouverture (610) entre le clapet anti-retour (612) et la deuxième extrémité (606) du conduit d'inhalation d'adaptateur (630) prévue pour être en communication fluide avec le moyen (638) destiné à fermer la deuxième extrémité (608) du conduit d'exhalation d'adaptateur (622) pour fournir une pression de commande pour commander la pression expiratoire d'extrémité positive.

6. Adaptateur (600) selon la revendication 1, dans lequel la première extrémité (604) du conduit d'exhalation d'adaptateur (622) a une position relative fixe par rapport à la première extrémité (602) du conduit d'inhalation d'adaptateur (630), l'adaptateur (600) comprenant un moyen pour être connecté à un tuyau de ventilation comprenant à la fois le conduit d'inhalation de tuyau (628) et le conduit d'exhalation de tuyau (626).

7. Adaptateur (600) selon la revendication 6, dans lequel le moyen destiné à être connecté à un tuyau de ventilation (624) est prévu pour être connecté à un tuyau coaxial.

8. Adaptateur (600) selon la revendication 1, dans lequel le moyen (614, 638) destiné à fermer la deuxième extrémité (608) du conduit d'exhalation d'adaptateur (622) comprend une valve (638) pour fournir une fermeture pouvant être ouverte de la deuxième extrémité (608) du conduit d'exhalation d'adaptateur (622), dans lequel la deuxième extrémité (608) du conduit d'exhalation d'adaptateur (622) est chanfreinée (644) à l'endroit où elle atteint la valve (638).

9. Tuyau de ventilation (624) comprenant au moins un conduit d'inhalation de tuyau (628) et un conduit d'expiration de tuyau (626) séparé, le tuyau de ventilation (624) étant intégré avec un adaptateur (600) selon l'une quelconque des revendications précédentes.

10. Agencement de ventilation comprenant un respirateur (100), un tuyau de ventilation (624) comprenant au moins un conduit d'inhalation de tuyau (628) et un conduit d'exhalation de tuyau (626) séparé, le tuyau de ventilation (624) étant connecté au respirateur (100) au moyen d'un adaptateur (600) selon l'une quelconque des revendications 1 à 8.
